# EUROPEAN PATENT APPLICATION

(11) **EP 3 758 024 A1**
(43) Date of publication of application: **30.12.2020**
(21) Application number: 19182863.1
(22) Date of filing: 27.06.2019
(51) Int. Cl.: G16H 50/20

(54) **RECOMMENDING CONTENT ITEMS TO A USER**

(71) Applicant: Koninklijke Philips N.V., 5656 AG Eindhoven (NL)
(72) Inventor: HARMA, Aki Sakari, 5656 AE Eindhoven (NL); CUBA GYLLENSTEN, Illapha Gustav Lars, 5656 AE Eindhoven (NL); VAN WISSEN, Arlette, 5656 AE Eindhoven (NL)
(74) Representative: de Haan, Poul Erik

(57) **Abstract**

A two-phase recommendation system for a recommendation device, employing both an external recommendation process and an internal, to a recommendation device, recommendation process. In particular, a processing unit uses a first data file, which is modifiable by an external source, and a second data file stored in a memory unit to recommend one or more content items to a user. The first and second data files are stored in a memory unit of the recommendation device.

## Description

### FIELD OF THE INVENTION

The present invention relates to the field of recommendation systems, and in particular to systems suitable for recommending one or more content items to a user.

### BACKGROUND OF THE INVENTION

Personal health services that provide educational, motivating and commercial content are more effective if the content is personalized to the needs and interests of the user. Recommendation systems are a known method of tailoring content to the user's preferences. Different methods can be used for this, including those that employ techniques such as collaborative filtering, content-based filtering and population clustering/classification in order to recommend appropriate content items to a particular user.

An increasing trend in recommendation systems has been the exploitation of cloud computing systems, external to a particular "recommendation device", to generate the recommendations for the user. Typically, the cloud computing systems receive user-specific information from the recommendation device and use this information to identify recommended content items for a user. This may be performed, for example, by comparing this user-specific information to user-specific information of other users, and providing a first user-specific recommendation based on the similarity of the information (e.g. by recommending content items that other, similar users have accessed).

Alternative methods have focused upon providing recommendation systems within a recommendation device itself. The recommendation device may itself be a stand alone device or may form part of an existing device, such as a mobile/cellular phone, television or computer. In one example, the recommendation devices recommends content items based on descriptors of the content items and user-specific information stored within the recommendation device (e.g. which may identify similar content items previously accessed by the user).

There is an ongoing desire to improve recommendation systems in order to provide users with a better content match.

### SUMMARY OF THE INVENTION

The invention is defined by the claims.

According to examples in accordance with an aspect of the invention, there is provided a processing unit for a recommendation device, comprising at least a memory unit and the processing unit, for recommending one or more content items to a user.

The processing unit is adapted to: attempt to obtain, from an external source to the recommendation device, first update information for updating a first data file, comprising information identifying an expected user-preference for one or more content items, stored by the memory unit; in response to successfully obtaining first update information, update the first data file based on the first update information; obtain the first data file, a second data file, comprising user-specific information for influencing the recommending of the one or more content items, and recommendation data, identifying a plurality of possible content items for recommendation to the user, from the memory unit; and process the first data file, the second data file and the recommendation data using a content selection algorithm to recommend one or more of the content items, identified by the recommendation data, to the user.

The present invention provides a method of technically implementing a recommendation system within a recommendation device that maintains privacy of a user whilst enabling external sources (such as a cloud computing system or network) to influence the recommendation of content. Embodiments thereby increase a privacy of the user whilst maintaining a high level of recommendation accuracy and allowing desirable recommendation methodologies to be used (e.g. which may require high levels of processing power or a large amount of data, which might not be available to the recommendation device itself). In particular, the invention provides a way to practically implement a new hybrid recommendation system that can exploit a greater processing power and data availability external to the recommendation device, whilst maintaining the privacy of a user's internal or localized data.

The recommendation device may be a stand alone or dedicated piece of hardware or, preferably, forms part of existing hardware (e.g. in an existing mobile/cellular phone, television, computer, tablet and so on).

By updating a first data file, stored in the recommendation device, based on first update information obtained from an external source, the external source can have an influence on the recommending of the content item(s) without itself performing the processing on personal data. This improves a privacy of the user of the recommendation device without losing the ability to use external data (e.g. such as global trends or the like), processing power or externally available algorithms, which may influence the recommendation.

Moreover, the proposed invention further enables recommendations to be provided to a user even if the external source is unavailable, e.g. if a connection to or other attempt to access the external source fails, which improves an availability and accessibility of the recommendation process. Thus, the recommendation device may be able to effectively operate "off-line".

The first data file may therefore represent global trends or information related to data of a larger population and the second data file may contain localized information (specific to the user or device). A content item may, for example, be a recommended article for reading by the user, an advertisement, a video for viewing by the user, forum messages or threads and so on.

Information for influencing the recommending of the one or more content items (which the first and second data file may both contain) may, for example, comprise a parameter for a variable used in an algorithm for generated recommended content. Suitable algorithms for recommending content are well known in the prior art, and typically comprise one or more variables that influence which content items are recommended.

In some embodiments, the recommendation data may itself comprise the plurality of possible content items for recommendations to the user.

The second data file is preferably separate and distinct from the first data file and may be configured so that at least some of the second data file is inaccessible by the external source. Optionally, all of the second data file is (at least initially) inaccessible by the external source. The inaccessibility may, for example, be overridden by a user providing express permission, e.g. via a user interface.

By not allowing (a portion of) the second data file to be inaccessible by the external source, the second data file can be kept private with respect to the external data source, thereby improving a privacy of the user. In preferable embodiments, information contained in the second data file is not passed to the external source, or is only passed to the external source with the express permission of the user.

The second data file may thereby store privacy-sensitive information, which the user may not wish to be passed to external sources, whilst still enabling the privacy-sensitive information to be used when recommending a content item.

The first data file may identify a plurality of possible content items for recommendation to a user and provide, for each identified content item, information identifying an expected user-preference for the associated content item.

Preferably, the information identifying an expected user-preference for the associated content item comprises a numerical measure of the expected user-preference for the associated content item.

In embodiments, the second data file provides information on historical accesses of content items by the user, and the processing unit is adapted to update the second data file in response to a user accessing a content item. Thus, the second data file may effectively contain historical access data of the subject, i.e. local near term history of the subject.

The processing unit may be adapted to obtain a measure of a time or user dependent variable; obtain a third data file comprising information, responsive to the measure, for influencing the recommending of the one or more content items; process the measure and the third data file to generate time or user based recommendation information for the user, and process the time or user based recommendation information, the first data file, the second data file and the recommendation data to recommend the one or more content items, identified by the recommendation data, to the user.

The measure of a time or user dependent variable acts as contextual information, e.g. about a current state of time or the user, for influencing the recommendation of the content item(s). The third data file provides information for mapping the variable measure to recommendation information that can be used when recommending one or more content items to the user.

Preferably, the time or user dependent variable is specific to the user (e.g. a day of pregnancy or a number of steps taken by the user).

For example, the third data file may comprise one or more functions that receives, as input, the measure and outputs a time or user based recommendation information. For example, the third data file may comprise identifiers of one or more possible content items for recommendation to a user and provide, for each possible content item, a function for indicating a relevance of the content item to the user for different values for the measure of the time or user dependent variable (e.g. over a period of time).

The measure may be a measure of time or any other measure that is responsive to a passage of time (i.e. time-based information or a time-based measure). For example, the measure may be a current time, a day of a user's pregnancy or a number of days until a user's birthday. The measure is preferably specific or directed towards the user (i.e. is user specific).

In this way, the measure may be time information of the user, the third data file may comprise time specific information (i.e. information that relies upon a passage of time) and the time or user based recommendation data may be time based recommendation information.

Other examples for the measure include a measure of any user dependent or user controllable variable (i.e. a variable that the user has an influence over). This may, for example, comprise a number of steps taken by the user (e.g. so far during the day), a number of programs or applications open on the recommendation device, a number of daily calories consumed by the user (e.g. as provided by the user), a number of drinks drunk that day by the user, a weight of the user and so on.

The processing unit may be further adapted to attempt to obtain, from an external source to the recommendation device, second first update information for updating the third data file stored by the memory unit; and in response to successfully obtaining second first update information, update the third data file based on the second first update information.

In embodiments, the processing unit is further adapted to receive a user input responsive to the recommended one or more content items; and communicate user information, responsive to the user input, to the external source, so that the external source obtains the user for modifying the first update information. Passing a user reaction to the recommended one or more content items to the external source enables the first update information to be more personalized to the user, without significantly impacting on a privacy of the user.

There is also proposed a recommendation device for recommending one or more content items for a user. The recommendation device comprises the memory unit adapted to store the first data file, the second data file and the recommendation data and any processing unit herein described. Of course, where the (optional) third data file is used, the memory unit may be further adapted to store the third data file.

The recommendation device may further comprise a display adapted to display identifiers of the one or more recommended content items to the user.

The recommendation device may further comprise a user interface for receiving a user input indicating a user's selection of an identifier of a recommended content item, wherein the display is further adapted to display a user-selected content item to the user. Thus, the user can access a recommended content item. The user interface and display may form a single element (e.g. a touch-sensitive display).

The recommendation device may comprise a mobile device for handheld transportation by the user. Examples of suitable mobile devices include a mobile phone, a laptop, a tablet, a smartwatch and so on.

In any described embodiment, the content item may comprise textual information, and the content selection algorithm is adapted to recommend one or more content items for readers of textual information. The textual information may be directed towards pregnant users, and the readers of the textual information may comprise the pregnant users.

There is also proposed a (distributed) recommendation system, comprising any herein described recommendation device and an external source, such as a cloud processing unit, adapted to generate the first update information for the first data item stored by the memory unit of the recommendation device.

According to examples in accordance with an aspect of the invention, there is provided a method of recommending one or more content items for a user.

The method comprises: storing, in a memory unit of a recommendation device: a first data file comprising information identifying an expected user-preference for one or more content items; a second data file comprising user-specific information for influencing the recommending of the one or more content items; and recommendation data identifying a plurality of possible content items for recommendation to the user; attempting to obtain, from an external source to the recommendation device, first update information for updating the first data file stored by the memory unit; in response to successfully obtaining first update information, updating the first data file based on the first update information; obtaining the first data file, the second data file and the recommendation data from the memory unit; and processing the first data file, the second data file and the recommendation data using a content selection algorithm to recommend one or more of the content items, identified by the recommendation data, to the user.

The method may further comprise: obtaining a measure of time or a user dependent variable; obtaining a third data file comprising information, responsive to the measure, for influencing the recommending of the one or more content items; processing the measure and the third data file to generate time or user-based recommendation information for the user. The step of processing the first data file, the second data file and the recommendation data may comprise processing the time or user based recommendation information, the first data file, the second data file and the recommendation data to recommend the one or more content items, identified by the recommendation data, to the user.

There is also proposed a computer program comprising code means for implementing any described method when said program is run on a processing unit.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Figure 1 illustrates a recommendation system comprising a recommendation device having a processor according to an embodiment of the invention;
Figure 2 illustrates a time-based function for influencing the recommendation of content items according to an embodiment; and
Figure 3 illustrates a method according to an embodiment of the invention.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a two-phase recommendation system, employing both an external recommendation process and an internal (to a recommendation device) recommendation process. In particular, a processing unit uses a first data file, which is modifiable by an external source, and a second data file stored in a memory unit to recommend one or more content items to a user. The first and second data files are stored in a memory unit of the recommendation device.

In particular, it will be clear that the first/second (and possibly more) data files may contain any data suitable for influencing the output of a content selection algorithm. The invention relies on the understanding that a first data file is controlled by an external source (and stored on the recommendation device), whilst the second data file is controlled by the recommendation device itself (and is, at least partially, not available to the external source). This improves an efficiency of selecting content items for recommendation whilst maintaining a privacy of the user.

Concepts may be employed in any recommendation system, such as in an application for recommending articles or information for pregnant users of a mobile device, such as a mobile/cellular phone.

In the context of the present invention, a content item may be an article, information, an advertisement, a video, a news story, textual information, visual information, pictures, a web-link, audio information and so on.

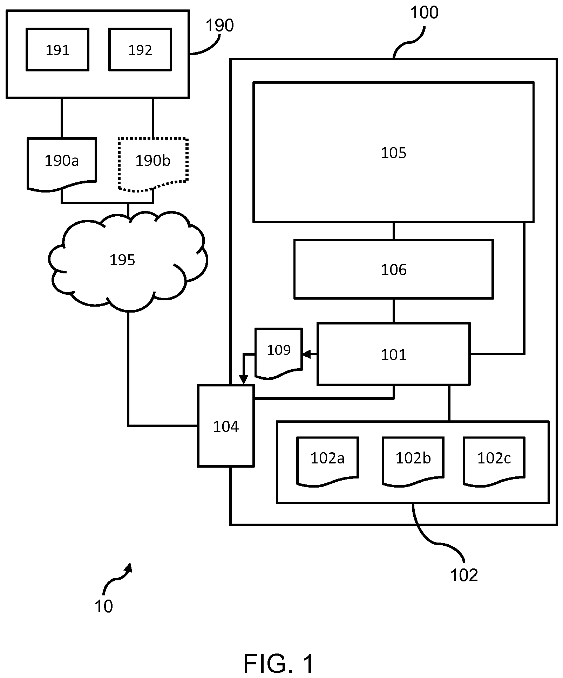

Figure 1 conceptually illustrates a recommendation system 10 according to an embodiment of the invention. The recommendation system 10 comprises a recommendation device 100 (by itself, an embodiment of the invention) and an external source 190, such as a cloud computing network or system. In Figure 1, data (files) are illustrated for the sake of improved conceptual understanding.

The recommendation device 100 is a device for direct interaction with a user and is preferably portable, such as a mobile phone, a tablet or a smart-watch. The recommendation device 100 recommends content items to the user.

The recommendation device 100 comprises a processing unit 101 (by itself, an embodiment of the invention) and a memory unit 102 adapted to store data files. The processing unit 101 and memory unit 102 (as well as other possible components of the recommendation device 100) are adapted to communicate with one another, for example, over a bus (not shown) or other communication link.

The processing unit 101 is adapted to recommend one or more content items for a user. This may be performed by the processing unit 101 selecting one or more content items from a known collection of content items using a recommendation or content selection algorithm. The processing unit 101 is adapted to recommend the one or more content items based on a first data file 102a, a second data file 102b and recommendation data 103c, all of which are stored in/by the memory unit 102.

The first data file 102a comprises information identifying an expected user-preference for one or more content items. Information stored in the first data file is effectively generated by or dependent upon information provided by the external source 190. In other words, the first data file is modifiable by the external source 190 and may represent an external sources recommendation of the content items.

The processing unit 101 is adapted to attempt to access the external source, e.g. via a communication module 104 of the recommendation device and a network 195, and obtain first update information 109a or data for updating the first data file 102a. In response to successfully accessing the external source, the processing unit 101 obtains first update information 109a and updates the first data file 102a. Thus, the first data file contains the most recently acquired information, for identifying an expected user-preference, generated by the external source 190. This allows the external source to have an influence on the recommendations to the user, thereby allowing potentially larger processing resources external to the recommendation device to influence the recommending process.

As previously explaining, the processing unit 101 may be adapted to communicate with the external source via a communication module 104 and a network 195 (e.g. comprising such as the Internet). The communication module 104, which may form part of the recommendation device 100, may be a wireless communication module adapted to operate using a WiFi standard or a mobile data communication standard (e.g. 3G, 4G and so on). In other examples, the communication module is adapted to enable wired communication between the external source 190 and the recommendation device. The network 195 comprises suitable element for enabling communication between the recommendation device 100 and the external source 190 (e.g. routers, modems, ISP servers and so on), as is well known in the art.

The processing unit 101 may be adapted to iteratively or periodically attempt to obtain the first update information 109a from the external source. The processing unit 101 may be unsuccessful in its attempt to obtain the first update information 109a, e.g. if the communication model is out of a wireless range or if the external source has an error.

The second data file 102b comprises user or device specific information that can influence the recommendation of content items. In one example, the second data file identifies content items previously accessed by the user, which may be used to influence the recommender to be less likely to recommend previously accessed content items. The second data file may identify other user or device specific information (e.g. type of device, brand of device, age of user, gender of user and so on) suitable for influencing the recommendation of content items.

Preferably, the second data file 102b is configured, e.g. using appropriate privacy or security settings, so that the external source 190 is unable to access or modify at least some of the data contained in the second data file 102b (e.g. even via the processing unit 101). This improves a privacy of the user of the recommendation device.

The processing unit 101 may therefore be configured to forbid the external source access to at least part of the second data file. This may be done by the processing unit 101 providing suitable privacy settings or security measures to store the second data file.

The second data file 102b can therefore store privacy-sensitive information, whilst still allowing said privacy sensitive information to contribute to the recommending of a content item.

The recommendation data 102c that identifies possible content items for recommendation by the processing unit. This may, for example, comprise a list identifying different possible content items (e.g. with descriptors for different content items) or a collection of the content items itself. Where the recommendation data 102c does not comprise the content items for recommendation, these may be stored elsewhere in the memory unit or in the external source.

The recommendation data 102c may be incorporated into one or both of the first 102a and second 102b data files.

Each of the first data file 102a, the second data file 102b and the recommendation data 102c can be accessed by the processing unit 101 even when access to the external source is not available. This enables the processor to generate recommendations even if the external source is not available.

It should be clear that the first and second data files are distinct and separate from one another, i.e. occupy different memory spaces within the memory unit 102.

As previously noted, the recommendation device 100 recommends one or more content items to a user, the recommended content items being identified by the recommendation data.

Identifiers of the recommended content items may be displayed to a user via a display 105 of the recommendation device 100. The processing unit 101 may control the display 105 to display the identifiers of the recommended content items. The display may thereby provide a content feed.

In some embodiments, the display 105 may be replaced by or supplemented with another user-perceptible output device, such as a speaker.

In some embodiments, the recommendation device further comprises a user interface 106 that enables a user to select a recommended content item. The user interface 106 may be integrated into a display 105 (e.g. a touch-sensitive display) or, as illustrated, be separate from the display (e.g. a keyboard and/or mouse input). The user interface 106 may generate a user input signal indicating the selected content item.

The processing unit 101 may be adapted to receive the user input signal and control the display to display the selected content item. This may be performed by retrieving the content item from the memory unit 102, or connecting to an external source 109 to retrieve the content item, and controlling the display to display the retrieved content item.

Preferably, the memory unit 102 stores available content items, i.e. "all possible content items", to improve an accessibility of the recommendation device 100. In particular, this enables off-line operation of the recommendation device. In such embodiments, the processing unit 101 can display a content item by retrieving a selected content item from the memory unit.

In some embodiments, the processing unit 101 is adapted to send user information 109 to the external source responsive to the user input signal. The user information 109 can be used by the external source to generate the first update information. In some examples, the user information identifies which content item(s) have been accessed by the user, e.g. responsive to their recommendation.

In some embodiments, the processing unit 101 is adapted to modify the second data file 102b responsive to the user input signal. For example, where the second data file provides information on historical accesses or views of the content items by the user, the processing unit may modify the second data file responsive to a user input signal indicating that a certain content item has been accessed or viewed.

In other embodiments, the user is not able to select a recommended content item, rather one or more recommended content items may be directly displayed or otherwise output to the user without direct user input. This is of particular benefit when the content item comprises an advertisement, which would not typically be selected by a user. The processing unit may still send user information 109 to the external source and/or update the second data file, but this action(s) may instead be responsive to the providing of the recommended content item to the user.

The recommendation device 100 is preferably a mobile or handheld device, such as a mobile phone, tablet or smartwatch. Other suitable examples of a recommendation device will be apparent, and may include a computer, laptop, smart speaker, television and so on.

Specific examples of suitable data files will now be described.

The first data file may comprise an expected preference table (EPT). The expected preference table may comprise identifiers of all possible content items (that a user can access or be presented with) and a score (e.g. between 0 and 1 or between 0 and 100) for each content item representing the expected preference of that content item for the user.

In one example, the first data file comprises a vector EPT[i] of scores, the index i of the vector identifying the content item. Thus, a first content item has a score at vector position of EPT[1], a second content item has a score at vector position EPT[2] and so on.

The "expected preference" is effectively a prediction by the external source of which content item would be most attractive to a user, and thereby a score indicating whether the external source believes that the associated content item should be presented/recommended to the user.

An expected preference table may be generated by the external source, e.g. a cloud computing system, using known recommendation algorithms. Effectively, the preference table may itself indicate which content items are recommended for the user (or group of users) by the external source.

The expected preference table may be a global preference table (e.g. indicating scores for all users), a demographic preference table (e.g. indicating scores for a group of users, including at least a user of the relevant recommendation device) or a user-specific preferable table (e.g. for the user of the relevant recommendation device). The external source may itself be adapted to generate one or more preference tables (e.g. a preference table for all users, each user or each of a group of users), of which one is obtained or selected by the recommendation device. Methods of generating a preference table, to be executed by the external source, will be briefly described later.

The first update information 190a may be an updated version of the first data file 102a or may comprise information for updating the first data file. In particular, the external source 190 may be adapted to generate a new version of the first data file and pass first update information to the processing unit 101 for updating the first data file, as would be well known to the skilled person.

The second data file may comprise, for example, a user reading history that indicates whether a user has interacted with (i.e. viewed or selected) a content item. The user reading history may comprise identifiers of all possible content items (e.g. as indicated by the recommendation data) and a value (e.g. 0 or 1; or 0 and 100) for each content item indicating whether the user has interacted with the relevant content item. For the following description the value '1' indicates that the user has not interacted with the content item and the value '0' indicates that the user has interacted with the content item, although other embodiments may vary.

The value within the user reading history may, in preferred examples, increase over time. Thus, after a user has accessed a particular content, the value associated with that content item in the user reading history may initially be 0 (indicating that the user has just accessed the relevant content item). This value may be incrementally increased over time (e.g. increased by 10% everyday), so that a user may be more likely to be recommended a content item that they haven't viewed for a longer period of time.

In another example, the value within the user reading history may switch from indicating that the content item has been viewed to indicating that the content item has not been viewed after a certain period of time (e.g. a day, a week or a month) has elapsed after the user viewed the content item. This would result in recently viewed content items being less likely to be shown to the user than less recently viewed content items.

In one example, the second data file comprises a vector URH[i] of values indicating whether the user has interacted with a content item, the index i of the vector identifying the content item, in a similar manner to the vector EPT[i] previously described.

It will be clear that the recommendation data may be formed as an aspect of the first data file and/or the second data file. For example, the indices of all possible content items in the first/second data file may represent the recommendation data.

The processing unit may use the first and second data file (at least one of which incorporates the recommendation data) to identify recommended content items. This is performed by processing said files using a content selection algorithm or method. Appropriate algorithms or methods of recommending content items are well known to the skilled person.

In a scenario in which the first data item comprises the vector EPT[i], described above, and the second data item comprises the vector URH[i] described above, one method of identifying a recommended content item includes performing the following equation: $s = {argmax}_{i} H \left(EPT, URH\right)$ (1) where s represents the index of the content item within EPT[i] or UGH[i], H(EPT, URH) is the Hadamard (element-wise) product of the vectors EPT, URH. The output of the Hadamard product is a vector ("Hardamard vector") and argmaxᵢ identifies the index (i.e. content item) corresponding to the element with the largest value in the Hardamard vector. One or more additional content item(s) can be recommended by subsequently identifying the indices (and therefore content items) associated with the next largest value in the Hardamard vector.

The operator H () may be replaced by other alternative linear or nonlinear operators that produce a vector of score values from a set of input vectors. In one possible embodiment, the operator H() comprises a machine-learning algorithm, such as a feedforward or recurrent neural network.

In this example, if the vector URH[i] provides a value 0 if an associated content item has already been viewed or accessed by the user and a value 1 if an associated content item has not already been viewed by the user, then equation (1) will not recommend a content item if it has already been viewed (as the value of the vector will be 0).

In some examples, recommendation data may correlate the index to a content item, which can be used to identify the content item (e.g. in the form of a look-up table). In a particular embodiment, the recommendation data may comprise all the content items and associate each content item with an index (matching the indices of EPT[i] and URH[i]).

In a further embodiment, the memory unit 102 further stores a third data file 102d. The third data file 102d comprises information for influencing the recommending of the one or more content items which, when processed with a measure of time or a user-dependent variable, generate a time or user based recommendation.

By way of example only, the third data file may comprise a look-up table comprising identifiers of all possible content items and, for each content item, a (time-variable) function that indicates a predicted interest in that content item for a subject based on a measure of time or a user dependent information, e.g. an elapsed time.

Hereafter, reference will be made to a time-variable function, for the purposes of improved understanding. However, the time-variable function may be replaced by any suitable function responsive to a user-specific variable.

The time variable function may be a Soft Applicability Model (SAM) function, which is a function that predicts how relevant an associated content item would be for the user at a particular moment in time. Typically, a(n) SAM function is a scaled and biased trapezoidal function of a temporal indicator, the scale and biasing of the function varying between different content items.

The time-variable function may, for example, receive time information as input and provide an output in the range of [0,1] or [0,100], the output representing a predicted interest in the content item for a time indicated by the time information. Time information may comprise any time-based measure or amount of time (e.g. a current time of day, an amount of time elapsed since a landmark event, an amount of time left until a landmark event is reached and so on). The amount of time may be measured in any suitable measure of time, such as days, hours, minutes, second, weeks, months, years and so on.

The time information may, for example, comprise a time of day. It is recognized that interest in different content items may vary over a time of day (e.g. there may be increased interest in breakfast-related content items in the morning and dinner-related content items in the evening).

In another example, the time information may comprise a day associated with a pregnancy of or associated with the user (i.e. a number of days since conception or a number of days until predicted due date). It is recognized that, over the course of a pregnancy, a user's interest in certain content items will vary. For example, early in the course of pregnancy, it may be assumed that a user may be more interested in alleviating symptoms associated with early pregnancy (e.g. so-called "morning sickness) compared to at an end of pregnancy. Similarly, towards an end of pregnancy, it may be assumed that the user would be more interested in content items associated with the birthing process or information on managing newborn babies, then during earlier in pregnancy.

Other suitable examples of time information will be apparent to the skilled person, e.g. a day of the year, a day of the month, an amount of time elapsed since or remaining until a public holiday, an amount of time elapsed since or remaining until a particular religious or festive day, an amount of time elapsed since or remaining until another landmark event (e.g. a user birthday, a birthday of a user's family member and so on), a stage between two landmark events associated with the user or a group including the user (e.g. a stage of the user's pregnancy, such as a current trimester, a stage of the user's education, e.g. a current school year/term/semester, a stage of the user's life, a stage of the year, e.g. spring, summer or winter, and so on), a time since the user last accessed a content item or a time since a user last accessed a particular application of the recommendation device (e.g. an application that performs the recommendation process).

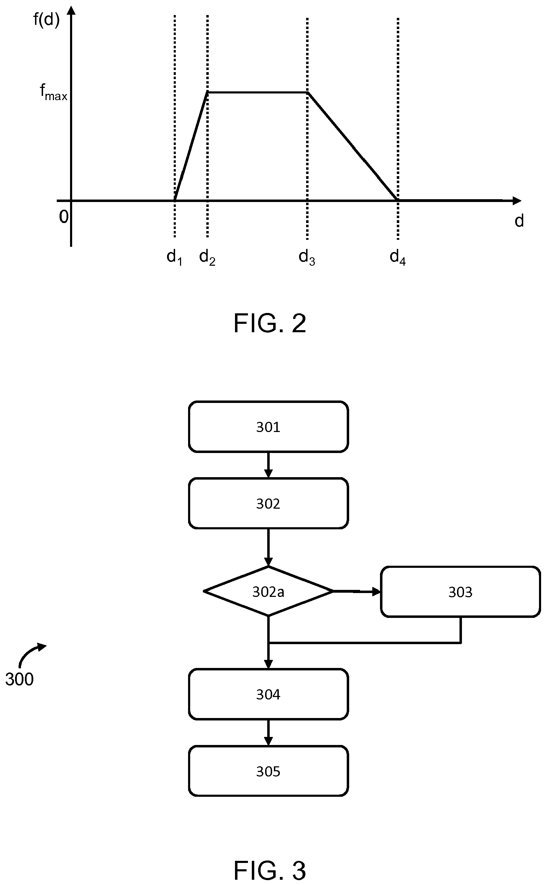

Figure 2 illustrates an example of a suitable time-variable function. The time-variable function provide a (continuous) output between 0 and 1, and is formed as a(n) SAM function.

The x-axis indicates the time information d, which here represents a day of pregnancy (e.g. a time elapsed since predicted conception, which may be provided by a user). The y-axis indicates the output of the time-variable function f(d), which provides a value representing (a modifier for) perceived or a predicted interest in that particular content item. The function f(d) is here a(n) SAM function. The value of the output of f(d) is, in Figure 2, always in the range of [0,1].

It will be observed that the SAM function f(d) provides a large value (i.e. closer to 1) when it is considered that the item is applicable for a user associated with particular time information d, (e.g. a certain point or phase during pregnancy). In Figure 2,

In the scenario of Figure 2, in which the time information d presents a day of pregnancy, it will be appreciated that a non-pregnant user might be associated with a day '0' (as they are not pregnant). At a time d₁ (e.g. a certain point during pregnancy) the value of f(d) rises before reaching a max value fₘₐₓ (which may be less than 1) at a time d₂. The value of the SAM function f(d) remains at this max value fₘₐₓ for a period of time, before beginning to fall at a time d₃. The value of the SAM function f(d) again reaches 0 at a time d₄.

The precise shape of the function f(d) may vary for different content items. In examples, the scale and bias (e.g. the position of points d₁ to d₄ and the max value) may be varied for different content items.

The SAM function may be effectively implemented as a vector SAM [f(d), i], where d is the time information (e.g. number of days since pregnancy), f(d) is the function defining an output value based on d, and the index i of the vector identifying the content item. The vector SAM [d, i] may identify all possible content items (i.e. the number of vector entries i is equal to the number of content items).

The processing unit may use the first, second and third data files (at least one of which incorporates the recommendation data) to identify recommended content items. Appropriate methods of recommending content items are well known to the skilled person.

In a scenario in which the first data item comprises the vector EPH[i], described above, the second data item comprises the vector URH[i] described above, and the third data item comprises the vector SAM [d, i] identified above, one method of identifying a recommended content item includes performing the following equation: $s = {argmax}_{i} H \left(EPT,SAM\left[f\left(d\right)\right],URH\right)$ (2) where s represents the index of the content item within EPT[i], UGH[i] and SAM[d], H(EPT, URH) is the Hadamard product of the vectors EPT, SAM[d], URH and argmaxᵢ identifies the index (i.e. content item) having the greatest Hadamard product. One or more additional content item(s) can be recommended by subsequently identifying the indices (and therefore content items) associated with the next highest Hadamard product.

As before, the function of equation 2 may be replaced using a machine-learning algorithm, such as a neural network.

The third data file may also be updated using second update information 190b obtained from the external source 190, in a similar manner to the first update information 190a. In some embodiments, a cloud computing system 191, of the external source 190, may provide the first update information and a content management system 192, of the external source 190, may provide the second update information 190b. In some embodiments, the cloud computing system may be adapted to generate the first update information responsive to the second update information.

It has previously been explained how the first update information may comprise replacement data for the first data file. The second update information may comprise replacement data for the third data file in a similar manner.

The external source 190, e.g. a cloud computing system 191, may generate the first/second update information using conventional collaborative filtering and content-based recommendation techniques.

One suitable method of generating first update information is hereafter described, in which the first update file and first update information both comprise an expected preference table (EPT) as previously set out. The first update file may reflect a most recently available copy of the first update information available to the processing unit.

In this method, each user (including at least the user of the recommendation device 100) is represented as a vector, such as a 10-dimensional vector, i.e. a "user vector". Each content item is represented by a vector of a similar dimension, i.e. a "content vector". The scalar product of these two vectors serves as the score predicting an expected preference of the user for that content item, i.e. the score indicating whether that content item should be presented/recommended to the user.

To find the vector representing the user, a minimization may be run over all the historical user reactions to content items (e.g. as passed to the external source via user information). In particular, historical reactions of the user to recommended content may be passed to the external device via user information. The external device may use these historical reactions to modify an initialized user vector so that a scalar product of the modified user vector and historic content vectors correctly predicts a reaction of the user to the historic content items (associated with the historic content vectors).

Elements of the historic content vector may, for example, identify a weighting of topics or themes within the associated historic content item.

Topics may relate to a specific subject area (e.g. "nutrition" or "sleep") and/or may relate to a style of the content item (e.g. "currently trending", "educational" or "industry approved") and/or a tone of the content item (e.g. "emphathetic" or "'authorative'"). Other methods of defining a topic will be known to the skilled person.

The external source may thereby be capable of generating a user vector that provides a representation of the user's preferred topics and themes. This user vector, when used, would thereby increase the influence of these topics and themes when generating a value that influences the recommending a content item.

Such methods of externally generating value indicative of recommendations are known to the skilled person. The present invention relies on the integration of such recommendation techniques in a more secure environment and without requiring connection to the external source, by passing external recommendations to a recommendation device (which stores said external recommendations). The external recommendations are then used by the recommendation device when recommended one or more of the content items.

The external source 190 thereby effectively operates as a "back-end" recommender, with the processing unit 101 operating as a "near-end" recommender. This effectively provides a two-phase recommendation system, employing both an external recommendation process and an internal (to a recommendation device) recommendation process.

The above embodiments have been described in the context of the first and second data files providing a score or value for each separate content item. However, in some embodiments, the first and second data files may be adapted to have a different influence on the content items.

By way of example, the first data file may indicate a user's predicted preference for certain topics, and the (separate) recommendation data may indicate topics associated with a particular content item. The processing unit may be adapted to more heavily weigh (for recommendation) content items associated with the topics identified in the first data file as forming part of the user's predicted preference.

By way of another example, the second data file may indicate a location of the user, and the (separate) recommendation data may indicate topics associated with particular locations. The processing unit may be adapted to more heavily weigh (for recommendation) content items associated with locations nearest to the location of the user (in the second data file).

By way of yet another example, the second data file may contain sensitive personal information, such as the stage of pregnancy, weight of the user, pregnancy weight gain measure or a medical condition influencing the pregnancy. The processing unit may be adapted to more heavily weigh (for recommendation) content items associated with the relevant stage/weight/condition of pregnancy.

Other suitable examples will be readily apparent to the skilled person.

Whilst only three data files have been described in the present embodiment, the processing unit may employ more than three data files when recommending content items.

Any herein described third data file may be used as a second data file. For example, the second data file may comprise time-specific information for influencing the recommendation of the one or more content items. Effectively, the second data file could be replaced by the third data file herein described.

In previous examples, a content item has been recommended by directly using the first and second data files to identify a content item for recommendation. In other embodiments, the recommendation is more indirect.

By way of example, the first and second data files may be processed to identify one or more topics for the user. The recommendation data (e.g. provided by the external source) may comprise a list of content items and topics associated with each content item, which enables a suitable content item to be selected (matching the identified one or more topics) and recommended to the user. The external source may be adapted to receive the identified one or more topics and identify the recommended content item.

It has previously been described how a machine-learning algorithm could be used to process the first data file, the second data file and the recommendation data (and optionally the third data file) to identify a content item for recommendation.

A machine-learning algorithm is any self-training algorithm that processes input data in order to produce or predict output data. Here, the input data the first data file, the second data file and the recommendation data (and optionally the third data file) and the output data comprises a content item for recommendation.

Suitable machine-learning algorithms for being employed in the present invention will be apparent to the skilled person. Examples of suitable machine-learning algorithms include decision tree algorithms and artificial neural networks. Other machine-learning algorithms such as logistic regression, support vector machines or Naive Bayesian model are suitable alternatives.

The structure of an artificial neural network (or, simply, neural network) is inspired by the human brain. Neural networks are comprised of layers, each layer comprising a plurality of neurons. Each neuron comprises a mathematical operation. In particular, each neuron may comprise a different weighted combination of a single type of transformation (e.g. the same type of transformation, sigmoid etc. but with different weightings). In the process of processing input data, the mathematical operation of each neuron is performed on the input data to produce a numerical output, and the outputs of each layer in the neural network are fed into the next layer sequentially. The final layer provides the output.

Methods of training a machine-learning algorithm are well known. Typically, such methods comprise obtaining a training dataset, comprising training input data entries and corresponding training output data entries. An initialized machine-learning algorithm is applied to each input data entry to generate predicted output data entries. An error between the predicted output data entries and corresponding training output data entries is used to modify the machine-learning algorithm. This process can repeated until the error converges, and the predicted output data entries are sufficiently similar (e.g. ±1%) to the training output data entries. This is commonly known as a supervised learning technique.

For example, where the machine-learning algorithm is formed from a neural network, (weightings of) the mathematical operation of each neuron may be modified until the error converges. Known methods of modifying a neural network include gradient descent, backpropagation algorithms and so on.

The training input data entries correspond to example first examples of the first data file, the second data file and the recommendation data (and optionally the third data file). The training output data entries correspond to content items for recommendation.

Figure 3 illustrates a method 300 of recommending one or more content items for a user.

The method comprises a step 301 of storing, in a memory unit of a recommendation device: a first data file comprising information identifying an expected user-preference for one or more content items; a second data file comprising user-specific information for influencing the recommending of the one or more content items; and recommendation data identifying a plurality of possible content items for recommendation to the user.

The method also comprises a step 302 of attempting to obtain, from an external source to the recommendation device, first update information for updating the first data file stored by the memory unit.

In response to successfully obtaining first update information, e.g. as determined in a step 302a, the method performs a step 303 of updating the first data file based on the first update information. Step 303 can be skipped if the first update information is not successfully obtained in step 302.

The method also comprises a step 304 of obtaining the first data file, the second data file and the recommendation data from the memory unit. The method further comprises a step 305 of processing the first data file, the second data file and the recommendation data using a content selection algorithm to recommend one or more of the content items, identified by the recommendation data, to the user.

The skilled person would be readily capable of adapting the method 300 to achieve any herein described concept or embodiment of the invention, e.g. as described with reference to Figures 1 and 2.

Similarly, the skilled person would also be readily capable of developing a processing unit for carrying out any herein described method. Thus, each step of the flow chart may represent a different action performed by a processing unit, and may be performed by a respective module of the processing unit.

Embodiments may therefore make use of a processing unit. The processing unit can be implemented in numerous ways, with software and/or hardware, to perform the various functions required. A processor is one example of a processing unit that employs one or more microprocessors that may be programmed using software (e.g., microcode) to perform the required functions. A processing unit may however be implemented with or without employing a processor, and also may be implemented as a combination of dedicated hardware to perform some functions and a processor (e.g., one or more programmed microprocessors and associated circuitry) to perform other functions.

Examples of processing unit components that may be employed in various embodiments of the present disclosure include, but are not limited to, conventional microprocessors, application specific integrated circuits (ASICs), and field-programmable gate arrays (FPGAs).

It will be understood that disclosed methods are preferably computer-implemented methods. As such, there is also proposed the concept of computer program comprising code means for implementing any described method when said program is run on a processing unit, such as a computer. Thus, different portions, lines or blocks of code of a computer program according to an embodiment may be executed by a processing unit or computer to perform any herein described method. In some alternative implementations, the functions noted in the block may occur out of the order noted in the figures. For example, two blocks shown in succession may, in fact, be executed substantially concurrently, or the blocks may sometimes be executed in the reverse order, depending upon the functionality involved.

In various implementations, a processor or processing unit may be associated with one or more storage media such as volatile and non-volatile computer memory such as RAM, PROM, EPROM, and EEPROM. The storage media may be encoded with one or more programs that, when executed on one or more processors and/or processing units, perform the required functions. Various storage media may be fixed within a processor or processing unit or may be transportable, such that the one or more programs stored thereon can be loaded into a processor or processing unit.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processing unit or other unit may fulfill the functions of several items recited in the claims. The mere fact that certain measures are recited in mutually different dependent claims does not indicate that a combination of these measures cannot be used to advantage. If a computer program is discussed above, it may be stored/distributed on a suitable medium, such as an optical storage medium or a solid-state medium supplied together with or as part of other hardware, but may also be distributed in other forms, such as via the Internet or other wired or wireless telecommunication systems. If the term "adapted to" is used in the claims or description, it is noted the term "adapted to" is intended to be equivalent to the term "configured to". Any reference signs in the claims should not be construed as limiting the scope.

## Claims

1. A processing unit for a recommendation device, wherein the recommendation device comprises at least a memory unit and the processing unit, the processing unit being suitable for recommending one or more content items to a user and being adapted to:
attempt to obtain, from an external source to the recommendation device, first update information for updating a first data file, comprising information identifying an expected user-preference for one or more content items, stored by the memory unit;
in response to successfully obtaining first update information, update the first data file based on the first update information;
obtain the first data file, a second data file, and recommendation data from the memory unit, wherein the second data file comprises user-specific information for influencing the recommending of the one or more content items and the recommendation data, identifies a plurality of possible content items for recommendation to the user; and
process the first data file, the second data file and the recommendation data using a content selection algorithm to recommend one or more of the content items, identified by the recommendation data, to the user.

2. The processing unit of claim 1, wherein the second data file is separate from the first data file and is configured so that at least some of the second data file is inaccessible by the external source.

3. The processing unit of any of claims 1 or 2, wherein the first data file identifies a plurality of possible content items for recommendation to a user and provides, for each identified content item, information identifying an expected user-preference for the associated content item.

4. The processing unit of any of claims 1 to 3, wherein each content item comprises textual information directed towards pregnant users, and the content selection algorithm is adapted to recommend one or more content items for pregnant users.

5. The processing unit of any of claims 1 to 4, wherein the second data file provides information on historical accesses of content items by the user, and the processing unit is adapted to update the second data file in response to a user accessing a content item.

6. The processing unit of any of claims 1 to 5, adapted to:
obtain a measure of time or a user controllable variable;
obtain a third data file comprising information, responsive to the measure, for influencing the recommending of the one or more content items;
process the measure and the third data file to generate time or user-based recommendation information for the user; and
process the time or user based recommendation information, the first data file, the second data file and the recommendation data to recommend the one or more content items, identified by the recommendation data, to the user

7. The processing unit of claim 6, wherein the third data file comprises identifiers of one or more possible content items for recommendation to a user and provides, for each possible content item, a function for indicating a relevance of the content item to the user for different values for the measure of time or a user controllable variable.

8. The processing unit of claim 6 or 7, further adapted to attempt to obtain, from an external source to the recommendation device, second update information for updating the third data file stored by the memory unit; and in response to successfully obtaining second update information, update the third data file based on the second update information.

9. The processing unit of any of claims 1 to 8, further adapted to:
receive a user input responsive to the recommended one or more content items; and
communicate user information, responsive to the user input, to the external source, so that the external source obtains the user for modifying the first update information.

10. A recommendation device for recommending one or more content items for a user, the recommendation device comprising the memory unit adapted to store the first data file, the second data file and the recommendation data and the processing unit of any of claims 1 to 9.

11. The recommendation device of claim 10, further comprising a display adapted to display identifiers of the one or more recommended content items to the user.

12. The recommendation device of any of claims 10 to 11, wherein the recommendation device comprises a mobile device for handheld transportation by the user, such as a mobile phone or tablet.

13. A recommendation system, comprising the recommendation device of any of claims 10 to 12 and an external source, such as a cloud processing unit, adapted to generate the first update information for the first data item stored by the memory unit of the recommendation device.

14. A method of recommending one or more content items for a user, the method comprising:
storing, in a memory unit of a recommendation device: a first data file comprising information identifying an expected user-preference for one or more content items; a second data file comprising user-specific information for influencing the recommending of the one or more content items; and recommendation data identifying a plurality of possible content items for recommendation to the user;
attempting to obtain, from an external source to the recommendation device, first update information for updating the first data file stored by the memory unit;
in response to successfully obtaining first update information, updating the first data file based on the first update information;
obtaining the first data file, the second data file and the recommendation data from the memory unit; and
processing the first data file, the second data file and the recommendation data using a content selection algorithm to recommend one or more of the content items, identified by the recommendation data, to the user.

15. A computer program comprising code means for implementing the method of claim 14 when said program is run on a processing unit.
